# EUROPEAN PATENT APPLICATION

(11) **EP 1 733 682 A2**
(43) Date of publication of application: **20.12.2006**
(21) Application number: 06252921.9
(22) Date of filing: 06.06.2006
(51) Int. Cl.: A61B 6/00, A61B 6/03

(54) **Imaging control method for X-ray CT apparatus**

(30) Priority: 08.06.2005 JP 2005168221
(71) Applicant: GE Medical Systems Global Technology Company, LLC, Waukesha, Wisconsin 53188-1696 (US)
(72) Inventor: Sasaki, Kosuke, Hino-shi Tokyo 191-8503 (JP); Sato, Kazuhiko, Hino-shi Tokyo 191-8503 (JP)
(74) Representative: Pedder, James Cuthbert

(57) **Abstract**

The present invention provides an imaging control method, which allows cardiac imaging of better quality to be obtained and an X-ray CT apparatus suitable for such imaging control. In order to control the start timing of the imaging by an X-ray CT apparatus, the method comprises computing the heart rate of the subject, and starting the imaging when the heart rate is stably settled. The heart rate computation is conducted on the heartbeats during the breath holding period. The start of imaging is automatic or manual when the heart rate stability is well fallen within the tolerance. The manual start of imaging is based on the indication that the heart rate stability is fallen within the tolerance. The manual start may also be based on the indication of the heartbeats or the transition of heart rate stability.

## Description

The present invention is directed to an imaging control method and an X-ray CT (computed tomography) apparatus, more specifically to a method of controlling the timing of start of imaging of the X-ray CT apparatus, and an X-ray CT apparatus for controlling the timing of start of imaging.

When imaging a heart by means of an X-ray CT apparatus, the heart is imaged while collecting the electrocardiogram signal, a predetermined number of view data around the desired view in the electrocardiogram phase is used to reconstruct an image. The imaging is conducted in helical scan or the like, while the patient is directed to hold the breath during imaging, and the data collection is conducted by an X-ray detector of multi slice type and the like (see for example JP-A-2004-275440 (pp. 3 & 5, Figs. 1, 2, 8, and 9)

In order to obtain a reconstructed image of better quality, it is necessary to image when the heart rate is stably settled. However the heart rate is in general start changing since the start of breath holding and is not well settled immediately. The time needed until the heart rate is settled and the extent of variation of heart rate is dependent on patients. An image of better quality cannot be obtained if the imaging is started at the same time that the breath is held.

Therefore, the present invention seeks to provide an imaging control method, which allows cardiac imaging of better quality, and an X-ray CT apparatus for controlling imaging as such.
(1) In an aspect of the present invention for solving the problems as have been described above, the present invention provides an imaging control method, for controlling the start timing of imaging by an X-ray CT apparatus, comprising the steps of: computing heart rate of a subject; and starting imaging when the heart rate is settled.
(2) In another aspect of the present invention for solving the problems as have been described above, the present invention provides an ray CT apparatus including an electrocardiogram signal collector device for collecting electrocardiographic signals of a subject, a data collector device for collecting data after imaging the subject by means of X-ray beam, a controller device for controlling the data collector device, and an image reconstruction device for reconstructing an image based on the data, the controller device comprising: a computer means for computing a heart rate based on the electrocardiogram signal; and a controller means for starting imaging when the heart rate is settled.

Preferably, the computation of heart rate is conducted on the heartbeats during a breath holding period, in order to prevent the breathing from affecting.

Preferably, the start of imaging is automatically conducted when the heart rate is settled within a predetermined range in order to automate the start of imaging.

Preferably, the start of imaging is manually conducted when the heart rate is settled within a predetermined range, in order to manually conduct the start of imaging.

Preferably, the manual start of imaging is conducted based on the indication that the heart rate is settled within the predetermined range, in order to facilitate the manual start of imaging.

Preferably, the manual start of imaging is conducted based on the indication of the transition of the heart rate or stability thereof, in order to facilitate the manual start of imaging.

Preferably, the stability is indicative of standard deviation of heartbeats in order to express the stability more precisely.

Preferably, the stability is indicative of differential of heartbeats in order to simplify the indication of stability.

The present invention in accordance with several aspects described above, starts imaging when the heart rate is settled by computing the heartbeats of the subject in order to control the start timing of imaging by the X-ray CT apparatus, allowing to achieve an imaging control method allowing cardiac imaging of better quality as well as an X-ray CT apparatus allowing control of imaging as such.

The invention will now be described in greater detail, by way of example, with reference to the drawings, in which:-
Fig. 1 shows diagrammatically a schematic block diagram of the X-ray CT apparatus in accordance with an exemplary embodiment of the best mode for carrying out the present invention.
Fig. 2 shows diagrammatically a schematic block diagram of the X-ray radiation and detection device in accordance with an exemplary embodiment of the best mode for carrying out the present invention.
Fig. 3 shows diagrammatically a schematic plan view of the X-ray incident plane of the X-ray detector in accordance with an exemplary embodiment of the best mode for carrying out the present invention.
Fig. 4 shows diagrammatically the transition of the heart rate during the breath holding period.
Fig. 5 shows diagrammatically the transition of standard deviation between heartbeats during the breath holding period;
Fig. 6 shows diagrammatically the transition of differential between heartbeats during the breath holding period.
Fig. 7 shows diagrammatically a schematic flow diagram of the operation of an exemplary X-ray CT apparatus in accordance with an embodiment of the best mode for carrying out the present invention.
Fig. 8 shows diagrammatically a schematic flow diagram of the operation of another exemplary X-ray CT apparatus in accordance with an embodiment of the best mode for carrying out the present invention.
Fig. 9 shows diagrammatically a schematic flow diagram of the operation of still another exemplary apparatus in accordance with an embodiment of the best mode for carrying out the present invention.

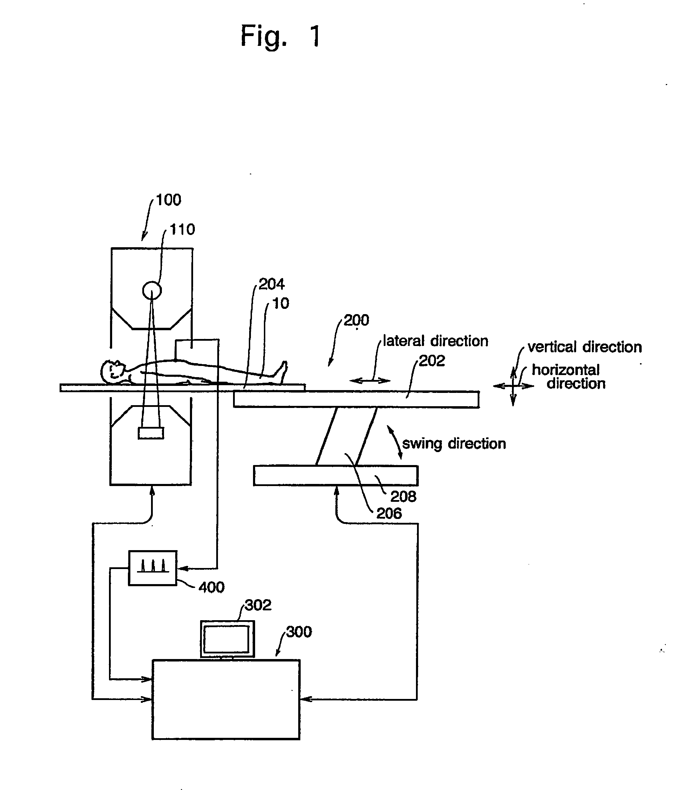

The best mode for carrying out the present invention will be described in greater details herein below with reference to accompanying drawings. It should be understood that the present invention is not intended to be limited to the best mode for carrying out the present invention disclosed herein. Now referring to Fig. 1, which shows diagrammatically a schematic block diagram of an X-ray CT apparatus. The apparatus is an exemplary embodiment for carrying out the present invention. The arrangement of the apparatus indicates an exemplary best mode for carrying out the present invention with respect to the X-ray CT apparatus. The operation of the apparatus indicates an exemplary best mod for carrying out the present invention with respect to the imaging control method.

As shown in Fig. 1, the apparatus includes a gantry 100, a table 200, an operator console 300, and an electrocardiograph 400. The gantry 100 images the subject 10 carried therein mounted on the table 200 by an X-ray radiation and detection device 110 to collect a plurality of views of data. The subject is imaged (scanned) by revolving the X-ray radiation and detection device 110 within the gantry 100. The gantry 100 is an exemplary embodiment of the data collector device in accordance with the present invention. The data collected by the gantry 100 is input to the operator console 300. The operator console 300 also receives the electrocardiogram signal from the electrocardiograph 400. The electrocardiograph 400 is an exemplary embodiment of the electrocardiogram signal collector device in accordance with the present invention. The operator console 300 incorporates a data processing unit such as for example a computer, and the input data and electrocardiogram signals are stored in its memory. The operator console 300 reconstructs an image based on the data and electrocardiogram signals. The operator console 300 is an exemplary embodiment of the image reconstruction device in accordance with the present invention.

The reconstruction of an image is conducted by means of data for a half scan or a full scan around the view of desired electrocardiogram phase. Thus reconstructed image will be displayed on a display 302.

The operator console 300 controls the gantry 100 and the table 200. Under the control of the operator console 300, the gantry 100 images in a predetermined imaging condition, while the table 200 positions the subject 10 in an imaging field so as to be able to scan a predetermined part thereof. The operator console 300 is an exemplary embodiment of the control device in accordance with the present invention. The operator console 300 is also an exemplary embodiment of the computing means and of the control means as well in accordance with the present invention.

The positioning of the subject 10 is performed by means of the built-in position adjustment mechanism, which adjusts the vertical height of the top plate 202, and the lateral displacement of a cradle 204 on the top plate 202. The height adjustment of the top plate 202 is performed by swingingly pivoting a stem 206 on the center of attachment to its base 208.

An axial scan can be conducted by scanning while the cradle 204 stops. On the other hand a helical scan is conducted by scanning with the cradle 204 continuously displaced.

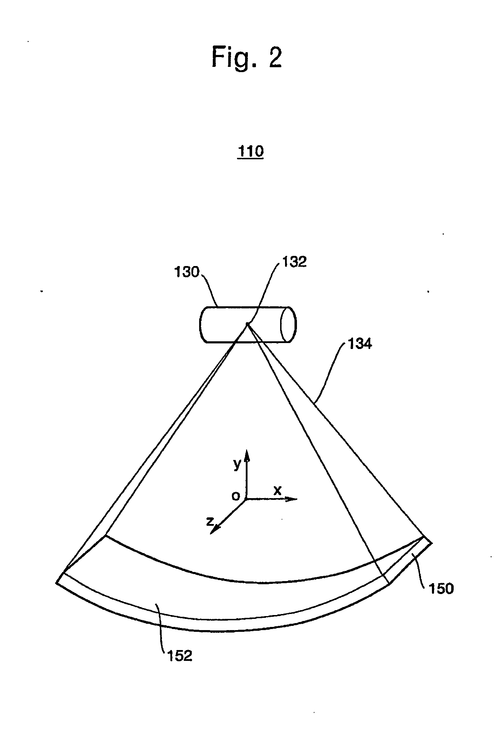

Now referring to Fig. 2, which shows diagrammatically a schematic block diagram of the X-ray radiation and detection device 110. The X-ray radiation and detection device 110 emits X-ray 134 from the focal point 132 of an X-ray tube 130 and detects the X-ray 134 with an X-ray detector 150. The X-ray 134 is shaped by a collimator not shown in the figure to a cone beam X-ray. The X-ray detector 150 has a two dimensional X-ray incident plane 152 in correspondence with the extent of the cone beam X-ray. The X-ray incident plane 152 is in the form of an arc, which constitutes part of a cylinder. The center axis of the cylinder passes through the focal point 132.

The X-ray radiation and detection device 110 revolves around the center axis passing through the image center or namely the isocenter O. The center axis is in parallel to the center axis of partial cylinder formed by the X-ray detector 150. By defining the direction of revolving center axis as z axis, the direction connecting the isocenter O with the focal point 132 as y direction, and the direction perpendicular to both z direction and y direction as x direction. The x-, y-, and z-axes are three axes of a revolved coordinate system around the center axis on z-axis.

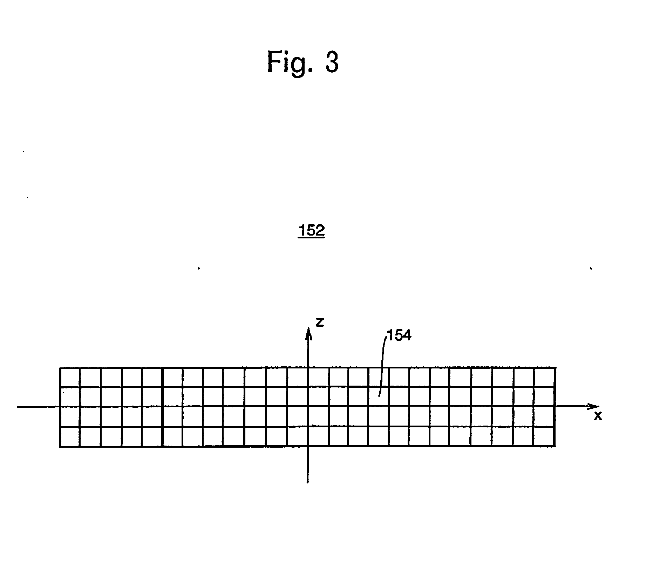

Now referring to Fig. 3, which shows diagrammatically a schematic plan view of the X-ray incident plane 152 of the X-ray detector 150. The X-ray incident plane 152 includes detector cells 154 in two dimension in the x direction and z direction. More specifically, the X-ray incident plane 152 is a two dimensional array of the detector cells 154.

Each of detector cells 154 forms a detector channel of the X-ray detector 150. The X-ray detector 150 thus is a multi-channel X-ray detector. The detector cells 154 may be formed by a combination of a scintillator and a photodiode.

The X-ray detector 150 as have been described above is also referred to as a multi-column X-ray detector or a multi-slice type X-ray detector. As the X-ray detector 150 is a multi-column X-ray detector, data for a plurality of slices can be obtained at once, allowing imaging of higher efficiency.

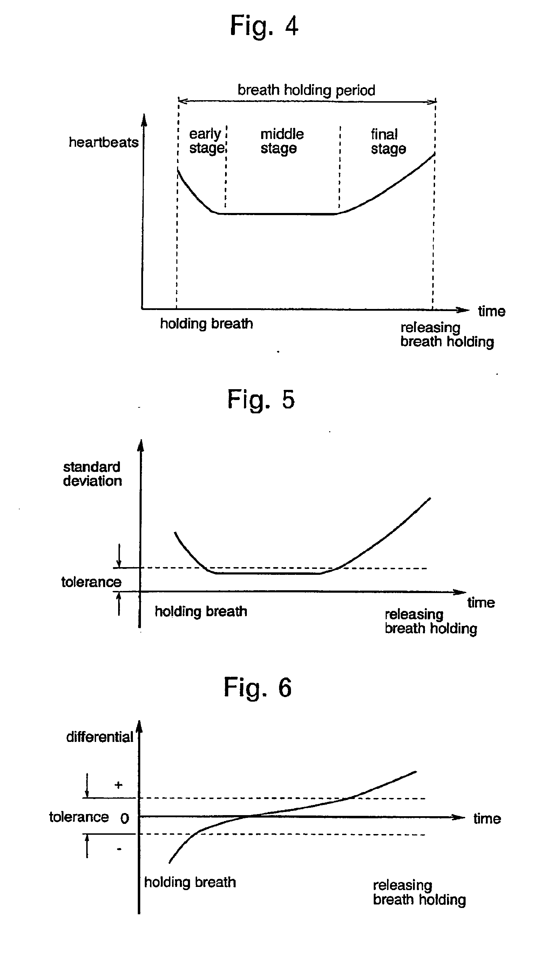

Cardiac imaging is conducted with the subject 10 holding the breath. The heart rate of the subject 10 during breath holding period transits in general as shown in Fig. 4. More specifically, at the beginning of the breath holding period the heart rate tends to decrease, at the middle of the period the heart rate is stable, and at the end of the period the heart rate tends to increase.

Now referring to Fig. 5, which shows diagrammatically the heart rate transition when indicating the transition of the heart rate in a standard deviation graph, the stable heart rate is shown by the decreased standard deviation. The standard deviation used here is the deviation of a plurality of heartbeats immediately adjoining. The combination of adjoining heartbeats is movingly altered. Using the standard deviation the stability of the heart rate can be precisely disclosed.

Alternatively, the stability of the heart rate may be indicated by the differential between heartbeats, instead of the standard deviation. The differential is a difference between the maximum heart rate and the minimum heart rate in successive adjoining plural heartbeats. The combination of successive adjoining plural heartbeats is movingly altered for each heartbeat. When indicating the transition of the heart rate as shown in Fig. 4, Fig. 6 for example is yielded, which indicates the stability of the heart rate by the decrease of differential. Using the differential facilitates to indicate the stability of the heart rate.

In view of the change in the heart rate as have been described above, it is preferable to conduct the imaging during the period in which the heart rate is settled. In the apparatus therefore, the timing of imaging is controlled so as to start imaging when the heart rate is stably settled. The control is performed in the operator console 300.

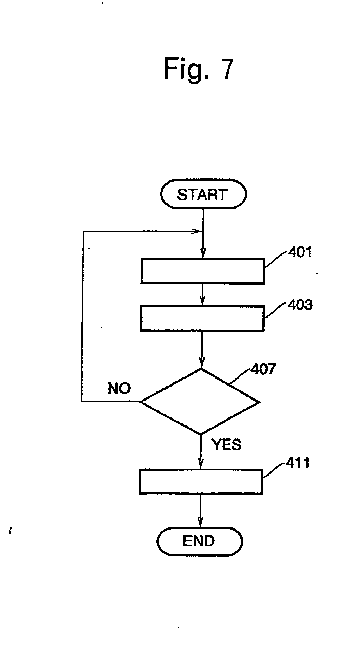

Now referring to Fig. 7, which shows diagrammatically a schematic flow diagram of the operation of an exemplary embodiment in accordance with the present invention. In step 401, the heart rate is computed. The computation of the heart rate is based on the electrocardiogram signals. More specifically, the reciprocal is solved from the period of electrocardiogram signals and is multiplied by 60 to yield the beat per minute (bpm).

In step 403, the stability of the heart rate is computed. As the index of stability of the heart rate, the standard deviation for example is computed. The differential between heartbeats may be computed instead of or in addition to the standard deviation.

In step 407, it is determined whether or not the stability of the heart rate is within the tolerance range. The tolerance of the heart rate stability is predefined. The upper limit of the tolerance in the standard deviation may be defined as 1 or 2. The upper and lower limit of the tolerance in the differential may be ± 1. If the heart rate stability is not within the tolerance, the process will proceed back to step 401. While the heart rate stability is not fallen within the tolerance, the system will iteratively repeat the operation of steps 401 to 407.

When the heart rate stability falls within the tolerance, then the imaging is conducted in step 411. More specifically, the operator console 300 issues an imaging instruction to the gantry 100, which, in response to the instruction, images the subject 10. Since the X-ray detector 150 is a multi-column X-ray detector which may obtain the data for a plurality of slices at once, the heart may be entirely scanned while the heart rate is being stably settled. In this manner, images of better quality can be obtained without being affected by the fluctuation of heart rate at the beginning of breath holding period.

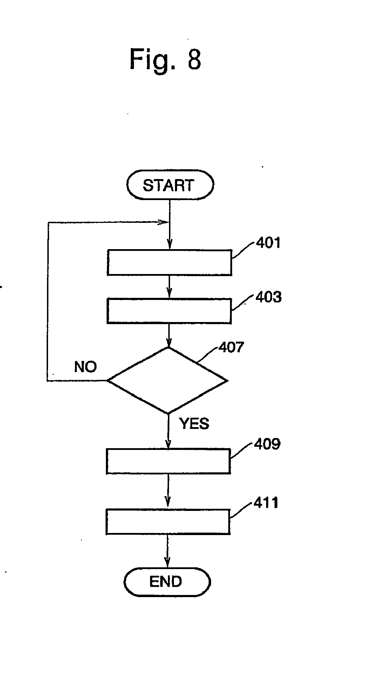

Now referring to Fig. 8, which shows diagrammatically a schematic flow diagram of the operation of another embodiment of the apparatus in accordance with the present invention. In step 401 the heart rate is computed, then the stability of the heart rate is computed in step 403. The standard deviation and/or differential as the indices of the heart rate stability are computed.

In step 407, it is determined whether or not the heart rate stability is fallen within the tolerance. If the heart rate stability is not within the tolerance then the process proceeds back to step 401. While the heart rate stability is not fallen within the tolerance, the process will iteratively repeat the operation of steps 401 to 407.

Once the heart rate stability falls within the tolerance, in step 409, an imaging button is lit. The imaging button is an illuminated operable button provided on the operator console 300. The operator is informed that the imaging is enabled when the imaging button is lit.

The operator pushes the button in correspondence with the notification, the subject is imaged in step 411. In these manner images of better quality can be obtained without being affected by the fluctuation of heart rate at the beginning of breath holding period.

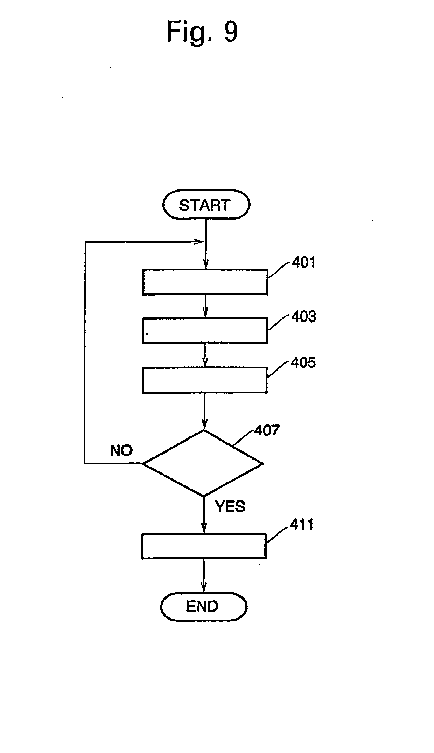

Now referring to Fig. 9, which shows diagrammatically a schematic flow diagram of the operation of another exemplary apparatus in accordance with the present invention. In step 401, the heart rate is computed, and the heart rate stability is computed in step 403. The standard deviation and/or differential as the indices of the heart rate stability are computed.

In step 405 the computation result of the heart rate stability is displayed. The result is displayed on the display 302. The contents of the display includes the transition of the standard deviation or differential values, a graph as shown in Fig. 5 or Fig. 6 is displayed along with the elapsed time. At this point the tolerance of the stability is also displayed. The item displayed on the display 302 may be altematively a graph indicative of the transition of the heart rate as shown in Fig. 4. The heart rate stability can also be recognized from this graph. In addition, by storing this graph in the memory, it can be used for the verification after the imaging.

In step 407, it is determined whether or not the heart rate stability is fallen within the tolerance range. The determination may be conducted by the operator monitoring the display 302. While the heart rate stability is not within the tolerance, the system will iteratively repeat the operation of steps 401 to 407.

The operator pushes the imaging button when he/she decides that the heart rate stability is well within the tolerance. Responsively the imaging will be conducted in step 411. In this manner, images of better quality can be obtained without being affected by the fluctuation of heart rate at the beginning of breath holding period.

Since the transition of the heart rate during the breath holding period may vary dependent on a personal error, it is preferable to recognize in advance the way of individual transition by conducting a dry rehearsal with the subject of breath holding to determine the standard deviation and the differential, in order to effectively run the imaging.

In case of imaging with the compensation for the body movement due to breathing, the breath holding during imaging session is not needed. However the imaging in that case requires the heart rate to be stably settled. The control of imaging timing in the manner as have been described above allows images of better quality to be obtained.

## Claims

1. An imaging control method, for controlling the start timing of imaging by an X-ray CT apparatus, comprising the steps of:
computing heart rate of a subject (10); and
starting imaging when said heart rate is settled.

2. An imaging control method according to claim 1, wherein said computation of heart rate is performed on the heartbeats during a breath holding period.

3. An X-ray CT apparatus, including an electrocardiogram signal collector device (400) for collecting electrocardiographic signals of a subject (10), a data collector device (100) for collecting data after imaging the subject (10) by means of X-ray beam, a controller device (300) for controlling said data collector device (100), and an image reconstruction device (300) for reconstructing an image based on said data, said controller device (300) comprising:
computer means for computing a heart rate based on said electrocardiogram signal; and
controller means for starting imaging when said heart rate is settled.

4. An X-ray CT apparatus according to claim 3, wherein:
said computation of heart rate is conducted on the heartbeats during a breath holding period.

5. An X-ray CT apparatus according to claim 3 or claim 4, wherein:
said start of imaging is automatically conducted when said heart rate is settled within a predetermined range.

6. An X-ray CT apparatus according to claim 3 or claim 4, wherein said start of imaging is manually conducted when said heart rate is settled within a predetermined range.

7. An X-ray CT apparatus according to claim 6, wherein said manual start of imaging is conducted based on the indication that said heart rate is settled within said predetermined range.

8. An X-ray CT apparatus according to claim 6, wherein said manual start of imaging is conducted based on the indication of the transition of said heart rate or stability thereof.

9. An X-ray CT apparatus according to any one of claim 5 to claim 8, wherein:
said stability is indicative of standard deviation of heartbeats.

10. An X-ray CT apparatus according to any one of claim 5 to claim 8, wherein:
said stability is indicative of differential of heartbeats.
